(19) Europäisches Patentamt
European Patent Office
Office européen des brevets

(11) EP 3 770 919 A1

(12) EUROPEAN PATENT APPLICATION

(43) Date of publication:
27.01.2021 Bulletin 2021/04

(51) Int Cl.:
*G16H 50/20* (2018.01)

(21) Application number: 19187680.4

(22) Date of filing: 22.07.2019

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **University College Dublin, National University of Ireland Dublin 4 (IE)**

(72) Inventors:
• **LOWERY, Madeleine**
  **Dublin 3 (IE)**
• **FLOOD, Matthew**
  **Co. Meath (IE)**
• **O'CALLAGHAN, Ben**
  **Co. Wicklow (IE)**

(74) Representative: **Barker Brettell LLP**
  **100 Hagley Road**
  **Edgbaston**
  **Birmingham B16 8QQ (GB)**

(54) **KINEMATIC DATA PROCESSING**

(57) A method (100) of determining the temporal occurrences of one or more events characterising movement of a person is provided. The method comprises processing (102) kinematic data relating to movement of the person to determine the temporal occurrences of the events. The processing of the kinematic data comprises processing the kinematic data using the Teager-Kaiser energy operator. A system for measuring the temporal occurrences of one or more events characterising movement of a person is also provided.

Fig. 1

- Record kinematic data using a kinematic sensor — 101
- Filtering data — 103
- Processing data using TKEO — 104
- Smoothing data — 105
- Peak detection — 106
- Processing kinematic data to determine temporal occurences of events — 102

100

**Description**

**Field of the Invention**

The invention relates to methods and systems for determining the temporal occurrences of one or more events characterising movement, for example hand movement, of a person using kinematic data.

**Background**

Finger tapping and other movement tasks are commonly used in the assessment of motor function in a number of movement disorders including Parkinson's Disease and Huntington's Disease. For example, movement disorder clinical rating scales such as the Unified Parkinson's Disease Rating Scale (UPDRS) or the Unified Huntington's Disease Rating Scale (UHDRS) include an assessment of motor function based on the performance of movement tasks such as finger tapping. Currently, the ratings are subjectively assessed by a clinician during a clinic visit and are therefore vulnerable to inter-rater variability and subjective results from clinicians. Assessing involuntary movements such as tremors, chorea and dyskinesias is also an important aspect of assessing motor function.

In order to reduce subjectivity, and when available, clinicians will perform video recordings of the movement disorder examination and a number of independent clinicians will perform the examination based on the video recordings. The raters must agree on a rating score within a given tolerance for the results to be considered valid. However, the use of video recordings in this way still has its drawbacks. Notably, the recording of videos increases the time burden on clinicians because it increases the time required for each clinical assessment and requires several clinicians to rate the performance of the movement task. Using video recordings without actual patient interaction may also skew results and certain tasks may not be possible to assess accurately using video only. Finding independent clinicians with no links to the patient so as to prevent subjective biases from impacting on the results of the examination is also challenging.

Alternatively, a number of different sensor types have been used to assess finger tapping task, such as gyroscopes, hall-effect magnetic sensors, touch sensors and accelerometers. These approaches use characteristics of the recorded signals to infer features of the finger tapping task such as the temporal occurrences of contact and release of the index finger and the thumb as well as quality of the movement during the task. However, these studies have not been validated using robust ground truth data and the accuracy of these methods is therefore questionable.

There is therefore a need for objective means of assessing the performance of movement tasks and the occurrences of involuntary movements that reduce inter-rater variability and subjectivity and allow clinicians to assess their patients in a standardised and controlled way that is cost-effective and time-efficient. There is also a need for a method of assessing the movements of those with movement disorders in a way that allows for a more in-depth analysis than is possible from visible inspection alone. There is also a need for a relatively non-intrusive and cost-efficient means of assessing the performance of movement tasks and the occurrences of involuntary movements that can be readily adapted to a number of different movement tasks and movements.

**Summary**

According to a first aspect of the invention, there is provided a method of determining the temporal occurrences of one or more events characterising movement of a person, for example that occur during a movement task performed by the person. The method may comprise processing kinematic data relating to or characterising movement of the person to determine the temporal occurrences of the events. The processing of the kinematic data may comprise processing the kinematic data using the Teager-Kaiser energy operator. Advantageously, the Teager-Kaiser energy operator enhances or accentuates high-frequency changes in the signal that correspond to the events.

Therefore, disclosed is use of the Teager-Kaiser energy operator in the processing of kinematic data relating to movement of a person to determine the temporal occurrences of one or more events that characterise the movement of the person.

The processing of the kinematic data may comprise performing a wavelet transform on the data before processing the kinematic data using the Teager-Kaiser energy operator. The processing of the kinematic data may comprise performing a multiresolution analysis on the data before processing the kinematic data using the Teager-Kaiser energy operator. The multiresolution analysis may be performed after the wavelet transform.

The processing of the kinematic data may comprise filtering the kinematic data, for example by high-pass filtering, prior to processing the kinematic data using the Teager-Kaiser energy operator.

The filtering of the kinematic data may comprise decomposing the kinematic data into a plurality of frequency levels. Each of the levels may comprise a subset of frequencies of the kinematic data. For example, each of the levels may comprise a plurality of wavelets characterised by different frequencies. Each of the levels may be characterised by a range or band of frequencies, each of the levels being characterised by a different range or band of frequencies. Each of the levels may be characterised by a centre frequency, the centre frequency of each of the levels being different. Each of the levels may act as a band-pass filter, each of the levels being characterised by a different centre frequency.

One or more of the levels of the filtered kinematic data may be processed using the Teager-Kaiser energy operator. The highest-frequency level of the filtered kinematic data may be processed using the Teager-Kaiser energy operator.

The filtering of the kinematic data may comprise performing a wavelet transform on the data. The wavelet transform may be a discrete wavelet transform. The discrete wavelet transform may be a maximal overlap discrete wavelet transform. The wavelet transform may use a Haar mother wavelet. The wavelet transform may decompose the kinematic data into a plurality of levels, for example as described above.

The filtering of the kinematic data may comprise performing a multi-resolution analysis. The multi-resolution analysis may decompose or reconstruct the kinematic data into a plurality of levels or series, as described above. One or more levels of the multi-resolution analysis may be processed using the Teager-Kaiser energy operator. The highest-frequency level of the multi-resolution analysis may be processed using the Teager-Kaiser energy operator.

The filtering of the kinematic data may comprise performing a wavelet transform and a multi-resolution analysis on the data.

The processing of the kinematic data may further comprise smoothing the kinematic data after processing using the Teager-Kaiser energy operator.

The processing of the kinematic data may further comprise identifying peaks in the kinematic data corresponding to the events following processing of the kinematic data using the Teager-Kaiser energy operator.

The temporal occurrences of the events may be determined based on the temporal locations of the peaks. For example, the temporal occurrences of the events may be determined as being the peak centres of the identified peaks.

The processing of the kinematic data using the Teager-Kaiser energy operator may comprise processing the kinematic data using the symmetric discrete time estimation of the Teager-Kaiser energy operator. For example, the processing of the kinematic data using the Teager-Kaiser energy operator may comprise calculating the symmetric discrete time estimation of the Teager-Kaiser energy operator for the data. Other expression or estimations of the Teager-Kaiser energy operator may alternatively be used.

The kinematic data may relate to movement of the person during a movement task. For example, the method may determine the temporal occurrences of one or more events that occur during a movement task.

The movement or movement task may comprise movement of at least a portion of one or both upper limbs of the person. The upper limbs may comprise the hands and arms of the person. For example, the movement or movement task may be or may comprise hand movement.

The movement task may be finger tapping. The finger tapping may comprise tapping a finger of one hand on the thumb of the same hand. The finger tapping may comprise tapping a finger of one hand on the thumb of the same hand in time to a metronome. The finger tapping task may comprise tapping a finger of one hand on the thumb of the same hand as often or as rapidly as possible as possible.

The events may comprise contact and/or separation (also known as release) of parts of the body of the person. For example, the events may comprise contact and/or separation a finger and a thumb of the person, for example if the movement task is finger tapping.

The kinematic data may be recorded by a kinematic sensor. The kinematic sensor may be an accelerometer worn by the person, for example during the movement task or movement. The kinematic data may be acceleration data, for example as recoded by the accelerometer. The kinematic sensor may be worn on a part of the person that moves during the movement task or movement. For example, the kinematic sensor may be worn on the upper limb of the person, more specifically on a finger (e.g. index finger) or thumb of the person. The kinematic sensor may be located at or near the tip of the finger to enhance sensitivity.

The determined temporal occurrences of the events may be used to generate an assessment of the motor function of the person. For example, the determined temporal occurrences of the events may be used to determine the motor function of the person according to a clinical rating scale. There is therefore disclosed a method of assessing motor function in a person. The method of assessing the motor function of the person may comprise processing kinematic data relating to movement of the person, for example during a movement task, to determine the timings of one or more events that characterise the movement, the processing of the kinematic data comprising processing the kinematic data using the Teager-Kaiser energy operator. An assessment of the motor function of the person may then be generated based at least on the determined temporal occurrences of the events.

The method of the invention may further comprise recording the kinematic data, for example using a kinematic sensor. The kinematic data may be recorded by a kinematic sensor. The kinematic sensor may be worn by the person during the movement or movement task. Alternatively, the step of recording the kinematic data need not be part of the method of the invention. As such there is disclosed a method of determining the temporal occurrences of one or more events in kinematic data relating to movement of a person.

Generally, summarising aspects of the invention recited above, there is disclosed a method of determining the temporal occurrences of one or more events characterising movement of a person, for example that occur during a movement task performed by the person, the method comprising: processing kinematic data relating to movement of the person, for example from a kinematic sensor worn by the person during a movement task, to identify the temporal occurrences of the events. The processing of the data comprises optionally filtering the data by high-pass filtering; then processing at least a portion of the data using the Teager-Kaiser energy operator; then optionally smoothing the data; and then optionally using at least one peak-detection algorithm to find peaks in the data corresponding to the events.

According to as second aspect of the invention, there is provided a system for measuring the temporal occurrences of one or more events. The events may characterise movement of a person. The events may occur during a movement task. The system may comprise a computer system configured to process kinematic data relating to the movement of the person to determine the temporal occurrences of the events. The processing of the kinematic data may comprise processing the kinematic data using the Teager-Kaiser energy operator.

The system may comprise a kinematic sensor for recording the kinematic data. The kinematic sensor may be a wearable kinematic sensor. In other words, the kinematic sensor may be configured to be worn by the person, for example on a part of the body that moves during the movement or movement task. The kinematic sensor may be or may comprise an accelerometer and the kinematic data may be or may comprise acceleration data.

According to a third aspect of the invention, there is provided one or more computer-readable storage media comprising instructions that, when executed by a computer, cause the computer to process kinematic data relating to movement of a person to determine the temporal occurrences of events characterising the movement of the person. The processing of the kinematic data may comprise processing the kinematic data using the Teager-Kaiser energy operator.

Also disclosed is a computer program comprising instructions that, when the program is executed by a computer, cause the computer to process kinematic data relating to movement of a person to determine the temporal occurrences of events characterising the movement of the person. The processing of the kinematic data may comprise processing the kinematic data using the Teager-Kaiser energy operator.

Features which are described in the context of separate aspects and embodiments of the invention may be used together and/or be interchangeable wherever possible. Similarly, where features are, for brevity, described in the context of a single embodiment, those features may also be provided separately or in any suitable sub-combination. Features described in connection with the method, system, computer readable medium/media and/or computer program may each have corresponding features definable and/or combinable with respect to each other, and these embodiments are specifically envisaged.

## Brief Description of the Figures

The invention will now be described, by way of example only, with reference to the following drawings, in which:

Figure 1 shows a flow diagram illustrating a method of determining the temporal occurrences of one or more events characterising movement of a person;

Figure 2 illustrates the experimental set-up used in the example;

Figure 3 shows the effect of the processing steps on the accelerometer data of the example;

Figure 4 presents histograms showing the distribution of the difference in contact times determined using the algorithm of the example vs actual contact times as determined by the touch sensors. FT1 corresponds to a metronome frequency of 0.5 Hz, FT2 corresponds to a metronome frequency of 0.625 Hz, FT3 corresponds to a metronome frequency of 0.833 Hz, FT4 corresponds to a metronome frequency of 1.25 Hz, FT5 corresponds to a metronome frequency of 2.5 Hz, and FT6 corresponds to unrestricted finger tapping as rapidly as possible; and

Figure 5 presents histograms showing the distribution of the difference in release times determined using the algorithm of the example vs actual release times as determined by the touch sensors. FT1 corresponds to a metronome frequency of 0.5 Hz, FT2 corresponds to a metronome frequency of 0.625 Hz, FT3 corresponds to a metronome frequency of 0.833 Hz,

FT4 corresponds to a metronome frequency of 1.25 Hz, FT5 corresponds to a metronome frequency of 2.5 Hz, and FT6 corresponds to unrestricted finger tapping as rapidly as possible.

**Detailed Description**

The Teager-Kaiser energy operator (TKEO) estimates the energy of a signal which is derived from the instantaneous amplitude and instantaneous frequency of the signal. Thus, it can be used to detect instantaneous changes in either amplitude or frequency in a given signal. The inventors have realised that, due to its ability to enhance high amplitude and frequency changes in signals, the TKEO is particularly useful in the determination of temporal occurrences, or timings, of events in movement tasks or of involuntary movements that are characteristic of movement disorders, which are often characterised by high amplitude and/or high frequency changes.

The invention therefore employs an algorithm that uses the TKEO to process kinematic data characterising movement of a person to identify, and to determine the temporal occurrences of, events characterising the movement of the person, for example hand movement. For example, the algorithm may be used to determine the temporal occurrences of events that occur during a movement task performed by a person. For example, the movement task may be finger tapping, which comprises tapping together one of the fingers on one hand, usually the index finger, and the thumb of the same hand. Specifically, the finger tapping task may comprise tapping the distal phalanx of the index finger and their thumb together while keeping their forearm and hand in the prone position. The finger tapping task may comprise tapping together the thumb and the finger at a given frequency, for example in time to a metronome, or may comprise tapping together the finger than the thumb as often or rapidly as possible. When the movement task is finger tapping, the events, which may be referred to as kinematic events, may be or may comprise the coming into contact of the finger and the thumb and/or the separation (also known as release) of the finger and the thumb.

Alternatively the movement task may comprise movement of any other part of the body. For example, the movement task may comprise movement of at least a portion of the upper limbs (including the arms and hands) of the person. For example, the movement or movement task may comprise hand movement. Typically, the events that are identified are characterised by high amplitude and/or frequency changes in kinematic data characterising the movement. For example, the events may comprise a rapid change of direction or an abrupt stop. The events are therefore generally associated with or are characterised by an increase, typically a rapid increase, in the instantaneous amplitude and/or frequency of the kinematic data, in particular the acceleration data.

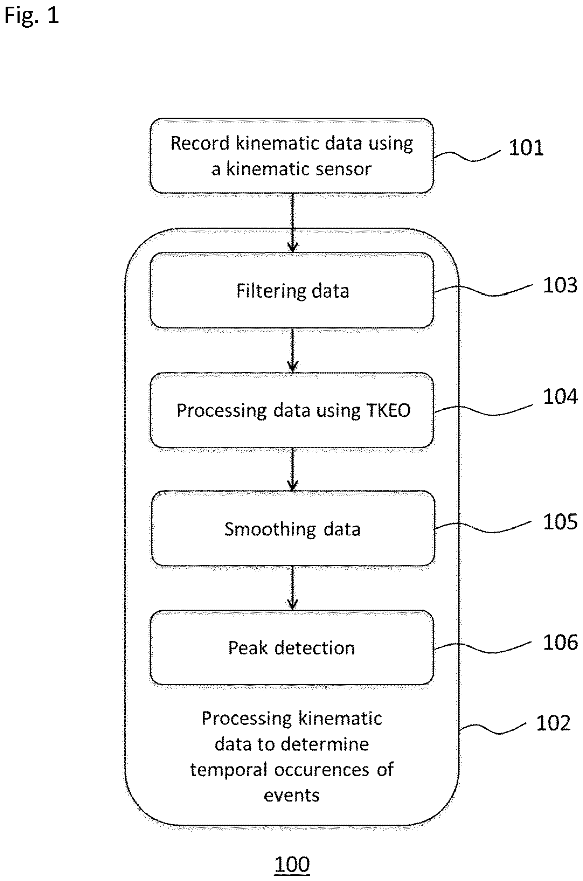

Referring to Fig. 1, a method 100 of determining the temporal occurrences of one or more events characterising movement of a person may optionally comprise the step 101 of recording kinematic data using a kinematic sensor. The kinematic data may be recorded while the person performs a movement task. Alternatively, the kinematic data may be recorded during a movement characteristic of or symptomatic of a movement disorder, such as tremor, chorea or dyskinesias. In particular, the kinematic data relates to or characterises movement of the person.

The kinematic data may be recorded by an accelerometer, specifically an accelerometer that is worn on the body of the person. The accelerometer may be a tri-axial accelerometer. The accelerometer should be worn on a part of the body that moves during the movement task or during the movements that are characterised by the events. For example, when the movement task is finger tapping the accelerometer may be worn on the finger or the thumb that are tapped together. Preferably, in this case, the accelerometer is worn on the finger that is tapped with the thumb, and the accelerometer is therefore configured to be worn on the finger. However, the accelerometer may be configured to be worn on another part of the body that moves during the movement or movement task. Advantageously, accelerometers provide a low-cost, lightweight and portable solution to accurately measure the complex movements of a body. However, a kinematic sensor other than an accelerometer may be used instead. The kinematic sensor used to record the kinematic data may, for example, be a gyroscope, a camera-based system, or may, for example, comprise a strain gauge. The kinematic data may be or may comprise acceleration data, particularly when the kinematic sensor is an accelerometer, but other kinematic data such as velocity may instead be used. The kinematic data may be the acceleration data of the major axis of motion, i.e. the axis in which the most movement occurs.

The method 100 comprises the step 102 of processing kinematic data relating to or characterising movement of the person, for example as obtained in step 101, to identify and determine the temporal occurrences, or timings, of events characterising or relating to movement of the person. Step 102 may comprise the step 103 of filtering the kinematic data. Specifically, the data may be high-pass filtered to remove or attenuate the lower frequency components of the data. This results in a relative enhancement of the high- or higher-frequency components of the data that are most useful in determining the temporal occurrences of the events. For example, the initial kinematic data may comprise high-frequency components and low-frequency components, and the filtering may enhance the high-frequency components in preference to the low-frequency components. The step of filtering of the kinematic data comprises decomposing the kinematic data into a plurality of levels or series, for example by performing a wavelet transformation on the data, each of the levels comprising a subset of frequencies of the kinematic data. Each of the levels may comprise a subset of frequencies of the kinematic data. For example, each of the levels may comprise a plurality of wavelets characterised by different frequencies. Each of the levels may be characterised by a range of frequencies, each of the levels being characterised by a different range of frequencies. Each of the levels may be characterised by a centre frequency, the centre frequency of each of the levels being different. Each of the levels may act as a band-pass filter, each of the levels being characterised by a different centre frequency. The first level contains the highest frequencies in the signal, with subsequent levels containing progressively lower frequencies.

Filtering step 103 may comprise performing a discrete wavelet transformation on the data. The discrete wavelet transformation may be the maximal overlap discrete wavelet transform (MODWT). The MODWT advantageously allows for easy alignment of the wavelet filtered data with the original time series, thereby maintaining temporal information. Specifically, the MODWT acts as a zero-phase filter, meaning that it doesn't distort the temporal information following the transform. Another advantage of the MODWT is that it allows for the use of a multi-resolution analysis (MRA), which can be used for a full reconstruction of the original data by summing all of the levels of the MRA. The discrete wavelet transformation may use a Haar mother wavelet, which advantageously accentuates high frequency and amplitude changes in the data.

Following the wavelet transform, filtering step 103 may comprise performing a multi-resolution analysis (MRA) on the data. This advantageously ensures zero-phase filtering of the signal and retains temporal resolution. The MRA results in the reconstruction of the original time series data into a sum of several new series or levels, each of which is related to variations in the data on a given time scale. The resulting levels may each correspond to a different frequency or range of frequencies, as described above in relation to the wavelet transform.

Processing step 102 comprises step 104 of processing at least a portion of the kinematic data using the TKEO. For example, a portion of the filtered kinematic data may be processed by the TKEO. This portion may comprise one or more of the levels of the data that results from the filtering, specifically one of the levels of the MRA. In particular, the first (highest-frequency) level of the filtered kinematic data may be processed using the TKEO. Using the first level is particularly advantageous because this corresponds to the highest frequency components of the data, which are generally the most useful in identifying the events, which also are also generally characterised by high-frequency and amplitude. In particular, using the highest frequency level provides particularly good results when identifying contact events between the finger and thumb during a finger tapping task, as demonstrated below in the example. However, other levels could be processed by the TKEO instead or in addition to the first level. For example, when identifying release events in finger tapping it may be advantageous to process using the TKEO a level that is not the first (highest frequency) level because the release events are generally lower frequency events than the contact events.

Processing the signal using the TKEO advantageously accentuates the high-frequency changes in the signal, which typically correspond to the events of interest, the events generally being associated with or characterised by high-frequency and/or amplitude changes in the data. As used herein, the term TKEO is intended to include the TKEO itself or other expressions of the TKEO, such as estimates of the TKEO. For example, in step 104 the data may be processed using the TKEO itself or a symmetric discrete time estimation of the TKEO rather than the TKEO itself. Other expressions, measures or estimates of the TKEO could alternatively be used in accordance with the invention.

Processing step 102 may optionally comprise smoothing step 105. Step 105 may comprise smoothing the data processed using the TKEO. The processed data may be smoothed using various established methods, for example using a moving maximum window followed by a moving mean window.

Processing step 102 may comprise peak detection step 106, either with or without smoothing step 105. Peak detection step 106 comprises using at least one peak-detection algorithm to find peaks in the data corresponding to the events. For example, in the case of finger tapping, a first peak-detection algorithm may first be applied to identify peaks corresponding to contact events, and a second peak-detection algorithm may then be applied to the data to search between the peaks found by the first peak detection algorithm to identify the peaks corresponding to the release events. The times, or temporal occurrences, of the events are then determined based on the temporal locations of the identified peaks in the data. The peak detection algorithm may be arranged to identify the local maxima within a signal. For example, only maxima with a temporal difference of at least 100ms and/or an amplitude greater than 5% of the maximum amplitude recorded in the data may be identified as peaks by the algorithm.

Step 102 may be implemented using one or more algorithms. For example, method 100 may comprise using one or more algorithms configured to perform one or more of steps 103 to 106, as described above.

Step 102 of method 100 may be performed by executing a computer program on a computer or computing device. The computer program may comprise instructions that, when the program is executed by a computer or computing device, cause the computer or computing device to process the kinematic data as described above to determine the temporal occurrences of the events. Similarly, one or more computer-readable storage media may be provided that comprise instructions that, when executed by a computer or computing device, cause the computer or computing device to process kinematic data relating to movement of a person to determine the temporal occurrences of events characterising the movement of the person, again as described above. In essence, the computer program or instructions are configured to perform some or all of the steps of the method described above, in particular those of step 102.

The determined timings, or temporal occurrences, of the events may be used to generate an assessment of the motor function of the person. For example, the determined temporal occurrences of the events may be used to determine the motor function of the person according to a clinical rating scale such as the Unified Parkinson's Disease Rating Scale (UPDRS) or the Unified Huntington's Disease Rating Scale (UHDRS). For example, the determined temporal occurrences of the events may be used in the calculation of the clinical rating according to the rating scale. This has the advantage that the motor skills of the person are determined without requiring a subjective assessment of the performance of a movement task, or the subjective assessment of involuntary movements such as tremors.

The invention also provides a system (e.g. a device or apparatus) for determining the temporal occurrences of events characterising movement of a person following the approach set out above. For example, the system may be configured to determine the temporal occurrences of events that occur during a movement task performed by a person. The system comprises a computer system configured to process kinematic data relating to movement of the person, for example during the movement task, to determine the timings of the events, as described above. For example, the computer system may comprise one or more processors and one or more computer-readable storage media comprising instructions that, when executed by the one or more processors, cause the processors to process kinematic data relating to movement of a person to determine the temporal occurrences of events characterising the movement of the person, again as described above.

The system may also comprise a kinematic sensor for recording the kinematic data, for example as described above. The kinematic sensor may be configured or arranged to be worn by the person, specifically on a part of the body of the person that moves during the movement task or during the movements that are characterised by the events. Specifically, the kinematic sensor may be configured to be worn on a finger, for example the index finger. The kinematic sensor may be an accelerometer, for example a tri-axial accelerometer. The data may be recorded by an accelerometer, specifically an accelerometer that is worn on the body of the person.

The system may comprise a wearable unit or device. The wearable device may be configured to be worn on a part of the body that moves during the movement task or during the movements that are characterised by the events, for example on a finger. The wearable device may comprise the kinematic sensor, and may optionally also comprise the computer system. Alternatively, the computer system may be separate from the wearable device. For example, the wearable device may communicate data to a separate computer system, for example a cloud-based computer system, that then processes the kinematic data. The wearable device may therefore be configured for wired or wireless communication for transmitting the data to a computer system for processing.

Example

The following describes a study demonstrating the effectiveness of processing accelerometer data using the TKEO in measuring the temporal occurrences of events in a movement task. In this case the movement task is finger tapping, and the events are the contact and release (or separation) of a finger and the thumb. Although this example serves to demonstrate the principles of the invention using finger tapping, the invention could equally be applied to other movement tasks or characteristic movements that are symptomatic of a movement disorder, in particular those that are characterised by events that are associated with high-frequency and/or large-amplitude changes in kinematic data.

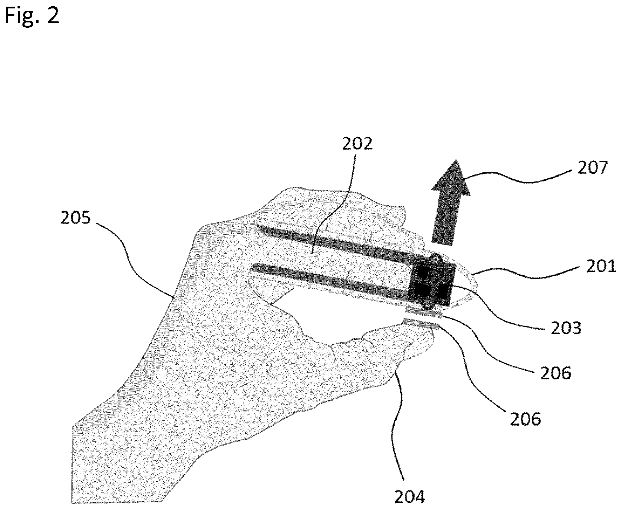

The subjects included 7 healthy, young participants (5 male, 2 female, age: 24.57 $\pm$ 1.51). Referring to Fig. 2, an aluminium splint 201 was placed over the index finger 202 of each of the subjects to restrict movement at the inter-phalangeal joints and was secured to the finger 202 with tape. A 3-axis digital accelerometer 203 (Grove ADXL345, 200 Hz) was then secured to the splint 201, specifically to the lateral aspect (side) of the splint 201, using tape. Subjects were instructed to tap the distal phalanx of the index finger 202 (or as close as possible) with their thumb 204 while keeping their forearm and hand 205 in the prone position. For the purposes of validation of the algorithm, touch or contact sensors 206 were applied to the contact surfaces of the index finger 202 and thumb 204 to provide a ground truth measure that accurately determined the occurrences of contact between the index finger 202 and thumb 204. The output of the contact sensors 206 was then compared to the output of the algorithm utilising the TKEO to assess the accuracy of the algorithm in determining the temporal occurrences (i.e. timings) of contact and release (i.e. separation) of the finger and the thumb.

Subjects were instructed to tap their index finger on their thumb in time with a metronome for a 30 second period. The metronome was set to five different frequencies (0.5, 0.625, 0.833, 1.25, 2.5 Hz). A final sixth trial consisted of un-queued tapping where subjects were instructed to tap as fast (often) as possible without a metronome beat to follow.

To calculate the temporal occurrences of the contact and release events of the index finger and thumb during the finger tapping task, a combination of high-pass filtering using the maximal overlap discrete wavelet transform (MODWT) and multi-resolution analysis (MRA) as well as the detection of simultaneous changes in frequency and amplitude of the signal using the TKEO algorithm were implemented in an algorithm comprising the following steps:

1) The raw accelerometer signal from the major axis of motion (denoted by arrow 207 in Fig. 2) was filtered using a maximal overlap discrete wavelet transformation with a Haar mother wavelet. The Haar wavelet was chosen for its ability to accentuate high frequency and amplitude changes in a signal.

2) Following the wavelet transform, a multi-resolution analysis was performed to ensure zero-phase filtering of the processed signal to retain temporal resolution. The MODWT acts as a series of zero-phase filters over the signal.

One very useful property of the MODWT is that it can be used to perform a multi-resolution analysis (MRA). A multi-resolution analysis allows for the reconstruction of the original time series signal into a sum of several new series, each of which is related to variations in the original signal at a given scale.

3) The first level (highest-frequency level) of the multiresolution analysis was then processed using the TKEO to further accentuate the high-frequency changes in the signal which corresponded to the contacts of the index finger and the thumb. In this example a symmetric discrete time estimation of the TKEO was used rather than the TKEO itself. Other expressions of the TKEO could alternatively be used. The following formula (1) was used to compute a symmetric discrete time estimation of the TKEO ($\Psi_n$) where $T_s$ is the sampling period, $x$ is the value of the signal and $n$ is the discrete (temporal) sample number (the data, i.e. the value of the signal, being expressed in discrete time samples n ordered sequentially in time):

$$\Psi_n = \frac{2x_n^2 + (x_{n+1} - x_{n-1})^2 - x_n(x_{n+2} + x_{n-2})}{4T_s} \tag{1}$$

4) The processed signal was then smoothed, in this case using a moving maximum window followed by a moving mean window.

5) A peak-detection algorithm was then employed to find peaks in the smoothened and processed signal which correspond to the contact events between the index finger and the thumb.

6) Once the contact events were identified, a further peak finding algorithm was used to search between the peaks associated with the contacts between the index finger and thumb to find the points of release (separation) of the index finger and the thumb.

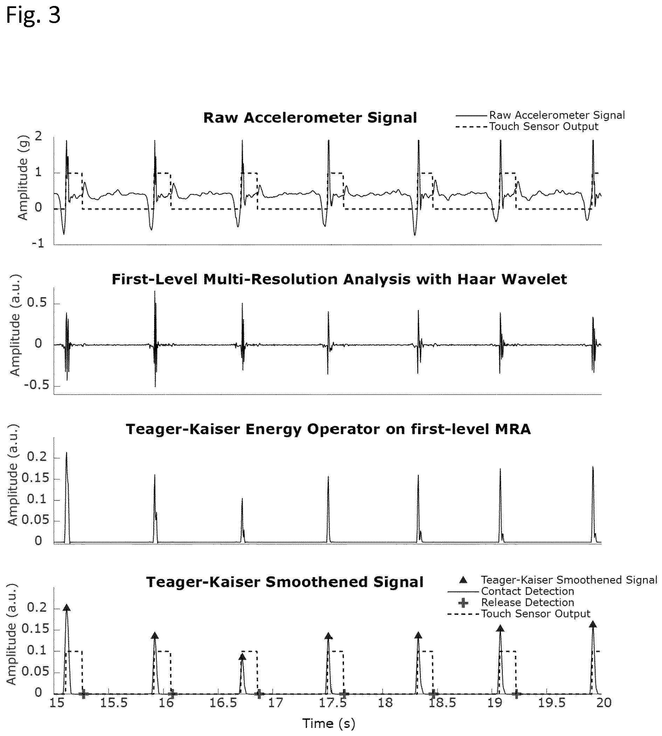

The temporal occurrences (i.e. timing) of the contact and release events were then determined based on the temporal locations of the identified peaks. The effect of this processing scheme on the accelerometer data is shown in Fig. 3.

The data recorded from the participants were analysed using MATLAB (The Mathworks Inc., Natick, MA, USA). Since the major axis of motion during the task was along the Y-axis of the accelerometer, the data from this axis was used to detect the events of the finger tapping task.

The contact times detected by the touch sensor were then compared to the times calculated by the algorithm by calculating the mean error, sensitivity, specificity and accuracy.

The mean absolute error was calculated as in equation (2) below:

$$MAE_{taps} = \sum_{k=1}^{k=N} | actual_k - detected_k | \tag{2}$$

where N is the total number of contacts between the index finger and thumb in the given recording, *actual* indicates the contact as determined by the touch sensor and *detected* indicates the contact as determined by the algorithm.

Sensitivity was calculated as in equation (3) below

$$Sensitivity = \frac{TP}{(TP + FN)}\%. \tag{3}$$

True positives (*TP*) were contacts that were identified by both the detection algorithm and by the touch sensors. False negatives (*FN*) were contacts that were not detected by the algorithm but were detected by the touch sensors.

The mean absolute error and its standard deviation (in brackets) for both the contact and release times as well as the sensitivity of the detection algorithm are displayed in Table 1.

**Table 1**

| Frequency of Metronome (Hz) | Mean Absolute Error of Contact Times (ms) | Mean Absolute Error of Release Times (ms) | Sensitivity (%) |
|---|---|---|---|
| 0.5 | 15.0 (9.6) | 43.9 (61.7) | 100 |
| 0.625 | 14.9 (7.5) | 28.2 (36.5) | 100 |
| 0.833 | 16.4 (7.6) | 32.5 (40.7) | 99.43 |
| 1.25 | 15.3 (6.5) | 33.5 (46.2) | 99.25 |
| 2.5 | 14.9 (6.6) | 25.3 (35.4) | 99.06 |
| N/A | 14.2 (4.8) | 43.4 (31.3) | 99.02 |

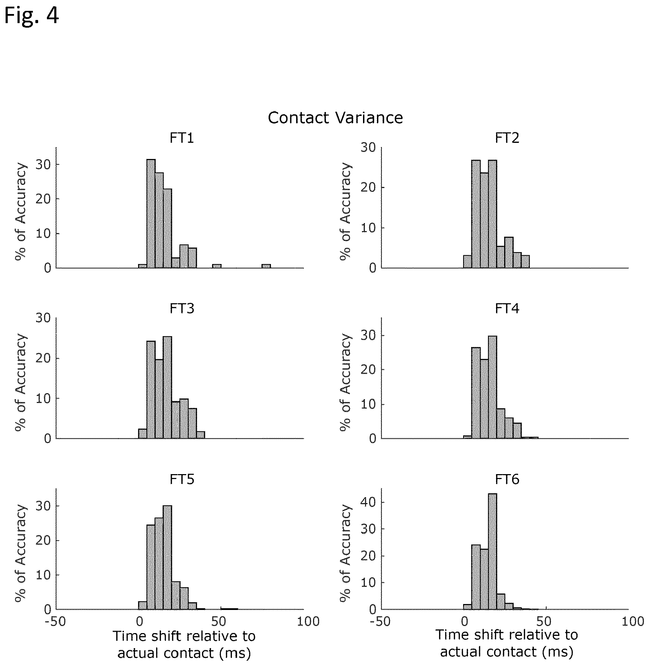

Fig. 4 displays histograms that show the time shift of the detected contact events relative to the actual contacts as determined by the touch sensors. It is clear to see that for all conditions, most of the contacts were identified within 50 ms of their actual occurrence.

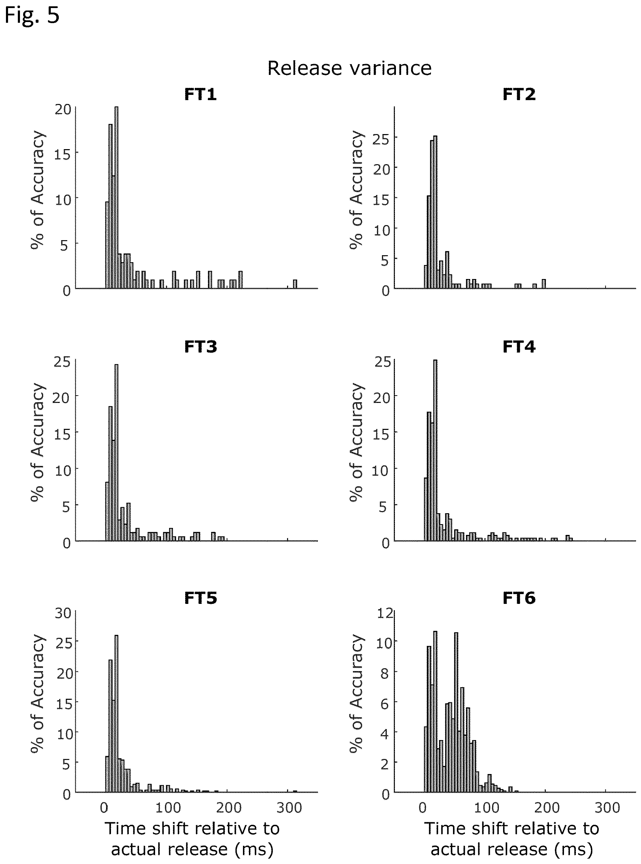

Fig. 5 displays histograms that show the time shift of the detected release events relative to the actual release events as determined by the touch sensors. It is clear to see that when tapping in time with a metronome most of the release events were identified within 50 ms of their actual occurrence, while most of the release events were identified within 100 ms of their actual occurrence when subjects performed finger tapping as rapidly as possible.

The results show that the algorithm is extremely sensitive to the finger tapping action as the sensitivity of the algorithm is greater than 99% for all conditions, with the lower tapping frequency conditions being the most sensitive. The mean absolute error of the algorithm is similar for all conditions, which suggests an inherent offset in the algorithm's detection of the contacts, which can easily be corrected for by shifting the measured contact times.

The accuracy of the algorithm in the measurement of the release times was lower, but was still high. The accuracy of the measurement of the release times may be improved by using a higher level (lower frequency) MRA signal because the opening action of the index finger and thumb is a relatively low frequency action. The algorithm in this case used the first level multi-resolution analysis signal. The first level MRA signal acts as a high-pass filter, passing only the high frequency components. Since the opening action of the index finger and thumb is a relatively low frequency action, it is more desirable to use a higher (lower frequency) level of the MRA analysis to detect these more accurately. The particular MRA level that will detect the release action most accurately will change depending on the frequency of the tapping, with higher frequencies being most accurately detected by lower (higher frequency) levels of the MRA signal and lower frequencies being most accurately detected by higher (lower frequency) levels of the MRA signal.

It will be appreciated by those skilled in the art that while the invention has been described above in connection with particular embodiments and examples, the invention is not necessarily so limited, and that numerous other embodiments, examples, uses, modifications and departures from the embodiments, examples and uses are intended to be encompassed by the claims attached hereto.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom. Features of the devices and systems described may be incorporated into/used in corresponding methods.

For the sake of completeness, it is also stated that the term "comprising" does not exclude other elements or steps, the term "a" or "an" does not exclude a plurality, a single processor or other unit may fulfil the functions of several means recited in the claims and any reference signs in the claims shall not be construed as limiting the scope of the claims.

## Claims

1. A method of determining the temporal occurrences of one or more events characterising movement of a person, the method comprising:

    processing kinematic data relating to movement of the person to determine the temporal occurrences of the events;
wherein the processing of the kinematic data comprises processing the kinematic data using the Teager-Kaiser energy operator.

2.  The method of claim 1, wherein the processing of the kinematic data comprises filtering the kinematic data by high-pass filtering prior to processing the kinematic data using the Teager-Kaiser energy operator.

3.  The method of claim 2, wherein the filtering of the kinematic data comprises decomposing the kinematic data into a plurality of levels, each of the levels comprising a subset of frequencies of the kinematic data;
optionally wherein one or more of the levels of the filtered kinematic data is processed using the Teager-Kaiser energy operator;
further optionally wherein the highest-frequency level of the filtered kinematic data is processed using the Teager-Kaiser energy operator.

4.  The method of claim 2 or 3, wherein the filtering of the kinematic data comprises performing a wavelet transform on the data;
optionally wherein the wavelet transform is a discrete wavelet transform;
further optionally wherein the discrete wavelet transform is a maximal overlap discrete wavelet transform.

5.  The method of any one of claims 2 to 4, wherein the filtering of the kinematic data comprises performing a multi-resolution analysis;
optionally wherein one or more levels of the multi-resolution analysis is processed using the Teager-Kaiser energy operator;
further optionally wherein the highest-frequency level of the multi-resolution analysis is processed using the Teager-Kaiser energy operator.

6.  The method of any preceding claim, wherein the processing of the kinematic data further comprises smoothing the kinematic data after processing using the Teager-Kaiser energy operator.

7.  The method of any preceding claim, wherein the processing of the kinematic data further comprises identifying peaks in the kinematic data corresponding to the events following processing of the kinematic data using the Teager-Kaiser energy operator;
optionally wherein the temporal occurrences of the events are determined based on the temporal locations of the peaks.

8.  The method of any preceding claim, wherein the processing of the kinematic data using the Teager-Kaiser energy operator comprises calculating the symmetric discrete time estimation of the Teager-Kaiser energy operator.

9.  The method of any preceding claim, wherein the kinematic data relate to movement of the person during a movement task and wherein the events occur during the movement task;
optionally wherein the movement task comprises movement of at least a portion of one or both upper limbs of the person;
further optionally wherein the movement task comprises hand movement;
further optionally wherein the movement task comprises finger tapping, and wherein the events comprise contact and/or separation of a finger and a thumb of the person.

10. The method of any preceding claim, wherein the kinematic data comprise acceleration data;
optionally wherein the acceleration data are recoded by an accelerometer worn on a part of the person that moves during the movement.

11. The method of any preceding claim, wherein the determined temporal occurrences of the events are used to generate an assessment of the motor function of the person.

12. The method of any preceding claim, further comprising recording the kinematic data.

13. A system for measuring the temporal occurrences of one or more events characterising movement of a person, the system comprising:

a computer system configured to process kinematic data relating to the movement of the person to determine the temporal occurrences of the events;

wherein the processing of the kinematic data comprises processing the kinematic data using the Teager-Kaiser energy operator.

14. The system of claim 13, further comprising a kinematic sensor for recording the kinematic data;

optionally wherein the kinematic sensor is a wearable kinematic sensor;

further optionally wherein the kinematic sensor comprises an accelerometer and the kinematic data comprise acceleration data.

15. One or more computer-readable storage media comprising instructions that, when executed by a computer, cause the computer to process kinematic data relating to movement of a person to determine the temporal occurrences of events characterising the movement of the person, wherein the processing of the kinematic data comprises processing the kinematic data using the Teager-Kaiser energy operator.

## Fig. 1

Record kinematic data using a kinematic sensor — 101

Filtering data — 103

Processing data using TKEO — 104

Smoothing data — 105

Peak detection — 106

Processing kinematic data to determine temporal occurences of events — 102

100

Fig. 2

## Fig. 3

Fig. 4

Contact Variance

## Fig. 5

Release variance

Europäisches
Patentamt

European
Patent Office

Office européen
des brevets

# EUROPEAN SEARCH REPORT

Application Number

EP 19 18 7680

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/206775 A1 (SARIA SUCHI [US] ET AL) 26 July 2018 (2018-07-26) * paragraphs [0011], [0012]; table II * | 1-15 | INV. G16H50/20 |
| X | MATTHEW WILLIAM FLOOD ET AL: "Gait Event Detection from Accelerometry using the Teager-Kaiser Energy Operator", IEEE TRANSACTIONS ON BIOMEDICAL ENGINEERING., 28 May 2019 (2019-05-28), pages 1-1, XP055659299, PISCATAWAY, NJ, USA. ISSN: 0018-9294, DOI: 10.1109/TBME.2019.2919394 * abstract * * p. 3, left-hand column, section "B. Comparative Algorithm Implementation" * | 1-15 | |
| X | US 2015/313509 A1 (HALKIAS XANADU CHRISTINA [US] ET AL) 5 November 2015 (2015-11-05) * paragraphs [0006], [0010], [0019], [0027], [0030] - [0035]; figure 2 * | 1-15 | |

TECHNICAL FIELDS
SEARCHED (IPC)

G16H

The present search report has been drawn up for all claims

| Place of search | Date of completion of the search | Examiner |
|---|---|---|
| Munich | 20 January 2020 | Heidrich, Alexander |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 19 18 7680

This annex lists the patent family members relating to the patent documents cited in the above-mentioned European search report.
The members are as contained in the European Patent Office EDP file on
The European Patent Office is in no way liable for these particulars which are merely given for the purpose of information.

20-01-2020

| Patent document cited in search report | | Publication date | Patent family member(s) | | Publication date |
|---|---|---|---|---|---|
| US 2018206775 | A1 | 26-07-2018 | NONE | | |
| US 2015313509 | A1 | 05-11-2015 | US 2013018283 | A1 | 17-01-2013 |
| | | | US 2015313508 | A1 | 05-11-2015 |
| | | | US 2015313509 | A1 | 05-11-2015 |

For more details about this annex : see Official Journal of the European Patent Office, No. 12/82